# EUROPÄISCHE PATENTSCHRIFT

(11) **EP 1 468 982 B1**
(45) Veröffentlichungstag und Bekanntmachung des Hinweises auf die Patenterteilung: **27.06.2012**
(21) Anmeldenummer: 04008181.2
(22) Anmeldetag: 03.04.2004
(51) Int. Cl.: C07C 69/34, C07C 69/38, C07C 69/40, C07C 69/42, C07C 69/44, C07C 69/48, C07C 69/50, C10M 105/34

(54) **Carbonsäureester auf Basis von 2-Hydroxymethylnorbornan**
Carboxylic acid esters on basis of 2-hydroxy-methyl-norbornane
Esters carboxyliques à base de 2-hydroxyméthyl-norbornane

(30) Priorität: 15.04.2003 DE 10317204
(43) Veröffentlichungstag der Anmeldung: 20.10.2004
(73) Patentinhaber: OXEA GmbH, 46147 Oberhausen (DE)
(72) Erfinder: Lappe, Peter, Dr., 46539 Dinslaken (DE); Springer, Helmut, 46539 Dinslaken (DE)

(56) Entgegenhaltungen:
- US-A- 2 912 458

## Beschreibung

Die vorliegende Erfindung betrifft neue Carbonsäureester auf Basis von 2-Hydroxymethylnorbornan, Verfahren zu ihrer Herstellung sowie ihre Verwendung

Carbonsäureester finden in großem Umfang und in vielfältiger Weise Anwendung in der Technik, wie zum Beispiel als Weichmacher, Schmiermittel und Riechstoffe. In der Industrie werden zahlreiche unterschiedliche Ester eingesetzt, ausgehend von einfachen Carbonsäureestern aus Monocarbonsäuren und Monoalkoholen bis hin zu komplexen Esterölen aus Mischungen von Mono- und Dicarbonsäuren mit mono- und polyfunktionellen Alkoholen. Durch die Auswahl geeigneter Ausgangsprodukte lassen sich die physikalischen Stoffeigenschaften, wie z.B. Siedepunkt oder Viskosität gezielt einstellen, und den chemischen Eigenschaften, wie z.B. der Hydrolysestabilität oder der Stabilität gegenüber einem oxidativen Abbau Rechnung tragen. Auch auf die Lösung konkreter anwendungstechnischer Probleme können Carbonsäureester gezielt zugeschnitten werden.

Ausführliche Übersichten über den Einsatz von Carbonsäureestern finden sich z.B. in Ullmann's Encyclopedia of Industrial Chemistry, 5. Auflage, 1988, VCH; Vol. A11, Seiten 191-193, Vol. A15, Seiten 438-440, Vol. A20, Seiten 439-458, Common Fragrance and Flavor Materials, Wiley-VCH 2001.

Die Verwendung von Carbonsäureestern als Schmierstoffe besitzt eine große technische Bedeutung. Der Begriff "Schmierstoffe" umfasst streng genommen nur Produkte, die für die Schmierung gleitender oder rollender Elemente verwendet werden. Die in der Technik in zahlreichen Anwendungen eingesetzten Schmierstoffe bestehen überwiegend aus Mineralölen oder volloder teilsynthetischen Produkten. Schmierstoffe auf Basis von Mineralölen sind sehr vielseitig anwendbar. Sie dienen nicht nur zur Schmierung und Kraftübertragung bei hohen und tiefen Temperaturen, sondern auch zur Wärmeübertragung und Isolierung. Für Anforderungen, die Mineralölprodukte nur unvollständig erfüllen, können synthetische Flüssigkeiten mit schmierölartigem Charakter zu technisch besseren Lösungen führen. Synthetische Grundöle werden aus weitgehend einheitlichen Substanzen unter kontrollierten Bedingungen hergestellt und können unterschiedlichen chemischen Verbindungsklassen angehören.

Eine besonders wichtige Verbindungsklasse stellen die Esteröle dar, diese werden in großem Stil beispielsweise im Flugverkehr als Turbinenmotorenund Instrumentenöle, als Fette oder Waffenöle verwendet. Diese Esteröle werden durch die Umsetzung von Säuren oder Säureanhydriden, insbesondere von Mono- oder Dicarbonsäuren und Alkoholen, insbesondere von Mono-, Di, Tri- oder Tetra-Alkoholen hergestellt.

Technisch wichtige Ausgangsprodukte für Säuren sind z.B. aliphatische Monocarbonsäuren mit 5-10 Kohlenstoffatomen. Als Dicarbonsäuren stehen beispielsweise Adipinsäure, Azelainsäure oder Sebacinsäure in technischen Mengen zur Verfügung. Als Alkohole kommen neben den aliphatischen Alkoholen wie 2-Ethylhexanol vor allem mehrwertige Alkohole wie Ethylenglykol und seine Oligomeren Di-, Tri- und Tetraethylenglykol, Propylenglykol und seine Oligomeren, Propandiol-1.3, Butandiol-1.4, Hexandiol-1.6, Neopentylglykol, Trimethylolpropan, Glycerin und Pentaerythrit zum Einsatz.

Aus US 2,912,458 ist die Veresterung des speziellen Alkohols 2-Methyl-2-norcamphanmethanols als Alkoholkomponente mit aliphatischen Dicarbonsäuren, insbesondere mit der Azelainsäure und der Sebacinsäure bekannt. Die hergestellten Esterverbindungen eignen sich als synthetische Schmiermittel. 2-Methyl-2-norcamphanmethanol wird durch Diels-Alder Reaktion von Cyclopentadien mit Methylmethacrylat, nachfolgender Hydrierung zur Carbonsäure und weiterer Hydrierung am Kupfer-Chromit-Katalysator hergestellt.

Die Entwicklung moderner Schmierstoffe und deren richtige Anwendung sind von erheblicher wirtschaftlicher Tragweite. Optimal der jeweiligen Aufgabe angepasste Schmierstoffe bringen durch Energieeinsparung, Verschleißminderung, verminderte Wartungszeiten und verlängerte Überholungsintervalle erhebliche Einsparungen. Daher besteht trotz der bereits zahlreich im Alltag und in der Technik eingeführten Produkte ein Bedarf an neuen Schmierstoffen mit verbesserten Eigenschaften.

Die Verwendung von Carbonsäureestern als Weichmacher besitzt ebenfalls eine große wirtschaftliche Bedeutung. Weichmacher finden in großem Umfang und in vielfältiger Weise Anwendung in Kunststoffen, Beschichtungsmitteln, Dichtungsmassen, Kautschuk- und Gummiartikeln. Sie treten mit hochpolymeren thermoplastischen Stoffen, ohne chemisch zu reagieren, vorzugsweise durch ihre Löse- und Quellvermögen in physikalische Wechselwirkung. Hierdurch bildet sich ein homogenes System aus, dessen thermoplastischer Bereich gegenüber den ursprünglichen Polymeren zu niederen Temperaturen verschoben ist, u.a. mit dem Ergebnis, dass seine mechanischen Eigenschaften optimiert, z.B. Formveränderungsvermögen, Elastizität, Festigkeit erhöht werden und die Härte verringert wird. Um Weichmachern möglichst weite Anwendungsgebiete zu eröffnen, müssen sie eine Reihe von Kriterien erfüllen. Im Idealfall sollten sie geruchslos, farblos, licht-, kälte- und wärmebeständig sein. Überdies erwartet man, dass sie unempfindlich gegenüber Wasser, schwer brennbar und wenig flüchtig sind und die Gesundheit nicht schädigen. Weiterhin soll die Herstellung der Weichmacher einfach sein und, um ökologischen Anforderungen zu genügen, unter Vermeidung von Abfallstoffen, wie nicht weiterverwertbaren Nebenprodukten und Schadstoffe enthaltenden Abwässern, erfolgen.

Zu den wichtigsten Weichmachern gehören die Ester von Di- und Polycarbonsäuren mit Weichmacheralkoholen, d.h. unverzweigten oder verzweigten primären Alkoholen mit etwa 6 bis 20 Kohlenstoffatomen, die als individuelle Verbindungen oder auch als Gemische eingesetzt werden. Als Esterweichmacher werden insbesondere Ester der Adipinsäure, der Azelainsäure und der Sebacinsäure zur Weichmachung von PVC verwendet.

Eine spezielle Klasse von Esterweichmachern, die auch mit der Bezeichnung G-Ester abgekürzt werden, enthält als Alkoholkomponente Diole bzw. Etherdiole, nämlich Ethylenglykol, Diethylenglykol, Triethylenglykol und Tetraethylenglykol.

Wie schon bei den Schmierstoffen ist die Entwicklung von modernen Weichmachern, die für die jeweilige anwendungstechnische Fragestellung zugeschnitten sind, von erheblicher wirtschaftlicher Bedeutung. Der Bedarf an optimal der jeweiligen Aufgabe angepassten Weichmachern mit verbesserten Eigenschaften ist trotz der zahlreichen, am Markt eingeführten Produkte von großem Interesse.

Aufgabe der Erfindung ist es daher, Carbonsäureester bereitzustellen, die mit besonders gutem Erfolg als Schmierstoffe oder als Weichmacher eingesetzt werden können. Ebenfalls liegt der Erfindung die Aufgabe zugrunde, ein Verfahren bereitzustellen, das die Herstellung solcher Carbonsäureester aus leicht zugänglichen, in ausreichender Menge preisgünstig zur Verfügung stehenden Ausgangsprodukten erlaubt. Besonderer Wert wird in diesem Zusammenhang darauf gelegt, dass der Veresterungsprozess mit einfachen technischen Mitteln realisiert werden kann und keine aufwendigen oder spezialisierten Apparaturen erfordert.

Die vorliegende Erfindung betrifft Carbonsäureester der allgemeinen Formel 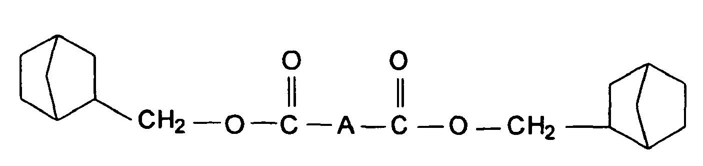 in der A gleich -(CH₂)ₓ-, mit x = 1 - 10 bedeutet.

Die Herstellung von 2-Hydroxymethylnorbornan als Alkoholkomponente erfolgt durch Hydroformylierung von Norbornen, einem kostengünstig und in großen Mengen zur Verfügung stehenden Olefin, das u.a. für die Produktion von Cycloolefin-Copolymeren technische Anwendung findet. Die unter Rh-Katalyse durchgeführte Hydroformylierung von Norbornen führt in sehr hohen Ausbeuten zum 2-Formylnorbornan. Die Umsetzung mit Synthesegas erfolgt im allgemeinen in homogener Phase in einem üblichen organischen Lösungsmittel, wie Cyclohexan, Toluol oder n-Hexan bei Temperaturen von 80 bis 150°C und Drücken von 10 bis 30 MPa in Gegenwart bekannter organischer Phosphor(III)-Verbindungen, wie zum Beispiel Triphenylphosphin, als Liganden. 2-Formylnorbornan wird durch destillative Aufarbeitung aus dem rohen Hydroformylierungsprodukt gewonnen. Anschließend wird 2-Formylnorbornan in Gegenwart üblicher Hydrierkatalysatoren mit Wasserstoff bei erhöhtem Druck und erhöhter Temperatur zum 2-Hydroxymethylnorbornan umgesetzt. Es lassen sich die in der Technik gängigen Hydrierkatalysatoren, wie zum Beispiel trägerhaltige oder trägerfreie Metallkatalysatoren, die beispielsweise Nickel, Palladium oder Kupfer als katalytisch aktives Metall enthalten, einsetzen. Darüber hinaus können gegebenenfalls Promotoren, wie Zirkon oder Mangan zugegen sein. Übliche Trägermaterialien sind Siliziumdioxid oder Aluminiumoxid.

Die Hydrierreaktion wird unter üblichen Temperaturbedingungen in einem Bereich von 70 bis 150°C und bei gängigen Druckbedingungen in einem Bereich von 2 und 30 MPa durchgeführt. Die Hydrierreaktion verläuft in hohen Ausbeuten. Damit steht 2-Hydroxymethylnorbornan nach einem technisch einfachen Verfahren kostengünstig für die Herstellung neuer Carbonsäureester zur Verfügung.

Als Dicarbonsäuren kommen vor allem die aliphatischen Vertreter - Malonsäure (x = 1), Bernsteinsäure (x = 2), Glutarsäure (x = 3), Adipinsäure (x = 4), Azelainsäure (x = 7), Sebacinsäure (x = 8) und 1.12-Dodecandisäure (x = 10) zur Anwendung. Diese einfachen Vertreter der aliphatischen Dicarbonsäuren sind in technischem Maßstab verfügbar oder können nach bekannten Verfahren hergestellt werden.

Die unmittelbare Veresterung von Alkoholen mit Carbonsäuren gehört zu den Grundoperationen der organischen Chemie. Um die Reaktionsgeschwindigkeit zu erhöhen, führt man die Umsetzung üblicherweise in Gegenwart von Katalysatoren durch. Der Einsatz eines der Reaktanten im Überschuß und/oder die Abtrennung des im Verlauf der Reaktion gebildeten Wassers stellt sicher, dass das Gleichgewicht entsprechend dem Massenwirkungsgesetz zur Seite des Reaktionsproduktes, also des Esters, verschoben wird, d.h. hohe Ausbeuten erzielt werden.

Für die Abtrennung des bei der Esterbildung gebildeten Reaktionswassers sind verschiedene Verfahren bekannt. Anwendung findet vorzugsweise die Azeotropdestillation in Gegenwart eines mit Wasser nicht mischbaren Lösemittels, das Erhitzen des Reaktionsgemisches unter Durchleiten eines inerten Gases, die Umsetzung der Ausgangsstoffe Alkohol und Carbonsäure unter Vakuum oder in Gegenwart eines Trocknungsmittels.

Insbesondere die Wasserentfernung durch Azeotropdestillation hat sich für die Einstellung des Gleichgewichts bei der Herstellung von Esterweichmachern bewährt. Üblicherweise setzt man als Azeotropbildner organische Lösemittel ein, die im technischen Maßstab preiswert zur Verfügung stehen. Geeignet sind aber auch alle anderen organischen Substanzen mit entsprechendem Siedepunkt, die mit Wasser Azeotrope bilden. Beispiele für eingesetzte Schleppmittel sind Hexan, 1-Hexen, Cyclohexan, Toluol, Benzol.

Die zur vollständigen Abtrennung des Wassers erforderliche Menge Schleppmittel lässt sich aus der entsprechend der Stöchiometrie der Veresterungsreaktion berechneten Wasserbildung und aus der Zusammensetzung des binären Azeotrops in einfacher Weise ermitteln. Es hat sich bewährt, das Schleppmittel im Überschuss einzusetzen, zweckmäßig in einem Anteil, der 50 bis 200 Gew.-% über der theoretisch berechneten Menge liegt. Durch Auffangen und Auftrennen des abdestillierten Schleppmittel/Wassergemisches lässt sich in einfacher Weise der Fortgang der Reaktion verfolgen. Das aus dem Azeotrop abgeschiedene Schleppmittel kann unmittelbar, d.h. ohne die Zwischenschaltung einer Reinigungsstufe, in die Reaktion zurückgeführt werden.

Die Reaktion von 2-Hydroxymethylnorbornan und Carbonsäure kann ohne Einsatz eines Katalysators durchgeführt werden. Diese Variante der Umsetzung hat den Vorteil, dass man vermeidet, dem Reaktionsgemisch Fremdstoffe zuzuführen, die zu einer unerwünschten Verunreinigung des Esters führen kann. Allerdings muss man dann im allgemeinen höhere Reaktionstemperaturen einhalten, weil nur so gewährleistet ist, dass die Umsetzung mit ausreichender, d.h. wirtschaftlich vertretbarer Geschwindigkeit abläuft. Zu beachten ist in diesem Zusammenhang, dass die Steigerung der Reaktionstemperatur zu einer thermischen Schädigung des Esters führen kann. Daher lässt sich nicht immer die Verwendung eines Katalysators, der die Umsetzung erleichtert und die Reaktionsgeschwindigkeit erhöht, vermeiden. Häufig kann der Katalysator ein Überschuß der Säure sein, die gleichzeitig Reaktionskomponente des 2-Hydroxymethylnorbornans ist. Im übrigen sind die üblichen Veresterungskatalysatoren zur Beeinflussung der Reaktionsgeschwindigkeit geeignet, wie Mineralsäuren, wie Schwefelsäure, Phosphorsäure, Polyphosphorsäure oder deren saure Salze, Trialkyl- oder Triarylphosphate, Ameisensäure, Methansulfonsäure oder p-Toluolsulfonsäure.

Die Menge des eingesetzten Katalysators kann sich über einen weiten Bereich erstrecken. Es können sowohl 0,01 Gew.-% als auch 5 Gew.-% Katalysator, bezogen auf das Reaktionsgemisch, verwendet werden. Da größere Katalysatormengen jedoch kaum Vorteile ergeben, beträgt die Katalysatorkonzentration üblicherweise 0,01 bis 1,0, vorzugsweise 0,01 bis 0,5 Gew.-%, jeweils bezogen auf das Reaktionsgemisch. Zweckmäßig entscheidet man gegebenenfalls durch Vorversuche für jeden Einzelfall, ob ohne Katalysator bei höherer Temperatur oder mit Katalysator bei niedriger Temperatur zu arbeiten ist.

Die Veresterung kann in stöchiometrischen Mengen an 2-Hydroxymethylnorbornan und Säure vorgenommen werden. Vorzugsweise setzt man jedoch 2-Hydroxymethylnorbornan im Überschuss ein, um in endlicher Zeit eine möglichst vollständige Umsetzung zu erreichen.

Die Reaktion zwischen 2-Hydroxymethylnorbornan und der Säure setzt in Abhängigkeit von den Einsatzmaterialien im Bereich von etwa 80 bis 140°C ein. Sie kann bei Temperaturen bis zu etwa 200°C zu Ende geführt werden. Bei diesen Temperaturangaben handelt es sich um Richtwerte, die zweckmäßig eingehalten werden. Niedrigere Temperaturen können z.B. ausreichen, wenn im speziellen Fall eine genügend hohe Reaktionsgeschwindigkeit erzielt wird oder nur Teilumsätze angestrebt werden. Höhere Temperaturen sind möglich, wenn das Auftreten von Zersetzungsprodukten, die u.a. farbschädigend wirken, auszuschließen ist. Die Anwendung von vermindertem oder erhöhtem Druck ist nicht ausgeschlossen, sie wird sich jedoch auf Sonderfälle beschränken.

Das nach beendeter Umsetzung anfallende Reaktionsgemisch enthält neben dem Ester als erwünschtem Reaktionsprodukt gegebenenfalls nicht umgesetzte Einsatzstoffe, insbesondere noch überschüssiges 2-Hydroxymethylnorbornan, sofern mit einem Alkoholüberschuss gearbeitet wurde. Zur Aufarbeitung wird der Reaktoraustrag nach konventionellen Verfahren vom Katalysator befreit. Liegt der Katalysator als Feststoff vor, z.B. in Form eines Hydrogensulfats, filtriert man das Produkt in üblichen Filterapparaten bei normaler Temperatur oder bei Temperaturen bis 150°C. Die Filtration kann durch gängige Filtrierhilfsmittel, wie Zellulose, Kieselgel, Kieselgur, Holzmehl unterstützt werden. Anschließend destilliert man überschüssige und nichtumgesetzte Ausgangsstoffe ab. Um letzte Reste acider Bestandteile zu entfernen, kann man auch noch eine Behandlung mit einem alkalischen Reagenz, z.B. wässriger Soda- oder Natriumhydroxidlösung vorsehen. Nach Phasentrennung wird das Rohprodukt fraktioniert destilliert. Sofern der Katalysator im Reaktionsgemisch gelöst vorliegt, wie Schwefelsäure oder para-Toluolsulfonsäure, destilliert man gegebenenfalls nach vorangegangener Filtration, zunächst noch vorhandene Ausgangsstoffe ab, behandelt daraufhin mit einem alkalischen Reagenz und fraktioniert den Rohester im Vakuum.

In einer weiteren Aufarbeitungsvariante wird nach Alkalibehandlung und Phasentrennung der Ester getrocknet, beispielsweise, indem man ein inertes Gas durch das Produkt leitet, Vakuum anlegt oder ein festes Trocknungsmittel, wie z.B. Natriumsulfat oder Magnesiumsulfat zusetzt, das nach erfolgter Trocknung abfiltriert wird. Gegebenenfalls unterzieht man das Produkt einer Wasserdampfdestillation vor dem abschließenden Trocknungsschritt.

Falls es der vorgesehene Verwendungszweck erfordert, können sich der Isolierung des Esters noch weitere Reinigungsschritte anschließen, beispielsweise eine fraktionierte Destillation im Vakuum.

Die Veresterungsreaktion kann absatzweise oder auch kontinuierlich in den für die chemische Technik typischen Reaktionsapparaten durchgeführt werden. Bewährt haben sich Rührkessel, die mit einer Heizvorrichtung sowie einer Einrichtung zum Zuführen des Azeotropbildners ausgestattet sind.

Die erfindungsgemäßen Ester eignen sich hervorragend als Weichmacher für alle gängigen hochpolymeren thermoplastischen Kunststoffe. Ebenso können sie als Schmierstoffe hervorragend verwendet werden.

Die folgenden Beispiele dienen zur Erläuterung der Erfindung, ohne sie darauf zu beschränken.

### Beispiel 1

### Herstellung von Di-(Norborn-2-yl-methyl)-malonsäureester

82,2 g (0,79 mol) Malonsäure, 218,3 g (1,73 mol) 2-Hydroxymethylnorbornan, 4,2 g (0,022 mol) p-Toluolsulfonsäure und 50 g Toluol werden in einem 11-Dreihalskolben mit Rührer, Innenthermometer und Wasserabscheider vorgelegt und zum Rückfluß erhitzt. Innerhalb von 2 Stunden werden 27,4 g Wasser ausgekreist. Das Reaktionsgemisch wird auf Raumtemperatur abgekühlt und mit 107,9 g wässriger Natronlauge (1 %ig) versetzt. Nach Phasentrennung verbleiben 311,9 g organische Phase, die anschließend mit 239,3 g Wasser gewaschen wird. Nach erneuter Phasentrennung wird die organische Phase (306,4 g) fraktioniert destilliert. Bei einer Kopftemperatur von 174°C und einem Druck von 100 Pa wird der Ester (233,3 g) in einer Reinheit von 96,3 % isoliert. Dies entspricht einer Ausbeute von 88,8 % der Theorie.

### Beispiel 2

### Herstellung von Di-(Norborn-2-yl-methyl)-bernsteinsäureester

Gemäß Beispiel 1 werden 93,3 g (0,79 mol) Bernsteinsäure, 218,3 g (1,73 mol) 2-Hydroxymethylnorbornan, 4,2 g (0,022 mol) p-Toluolsulfonsäure und 50 g Toluol zur Reaktion gebracht. Innerhalb von 2 Stunden werden 29,1 g Wasser ausgekreist. Das Reaktionsgemisch wird auf Raumtemperatur abgekühlt und mit 161,7 g wässriger Natronlauge (1 %ig) versetzt. Nach Phasentrennung verbleiben 321,0 g organische Phase, die anschließend mit 243,3 g Wasser gewaschen wird. Nach erneuter Phasentrennung wird die organische Phase (291,7 g) fraktioniert destilliert. Bei einer Kopftemperatur von 187°C und einem Druck von 100 Pa wird der Ester (237,5 g) in einer Reinheit von 98,1 % isoliert. Dies entspricht einer Ausbeute von 88,2 % der Theorie.

### Beispiel 3

### Herstellung von Di-(Norborn-2-yl-methyl)-glutarsäureester

Gemäß Beispiel 1 werden 104,3 g (0,79 mol) Glutarsäure, 218,3 g (1,73 mol) 2-Hydroxymethylnorbornan, 4,2 g (0,022 mol) p-Toluolsulfonsäure und 50 g Toluol zur Reaktion gebracht. Innerhalb von 2 Stunden werden 27,6 g Wasser ausgekreist. Das Reaktionsgemisch wird auf Raumtemperatur abgekühlt und mit 96,7 g wässriger Natronlauge (1 %ig) versetzt. Nach Phasentrennung verbleiben 358,0 g organische Phase, die anschließend mit 241,0 g Wasser gewaschen wird. Nach erneuter Phasentrennung wird die organische Phase (338,0 g) fraktioniert destilliert. Bei einer Kopftemperatur von 184 - 186°C und einem Druck von 100 Pa wird der Ester (241,6 g) in einer Reinheit von 98,7 % isoliert. Dies entspricht einer Ausbeute von 86,6 % der Theorie.

### Beispiel 4

### Herstellung von Di-(Norborn-2-yl-methyl)-adipinsäureester

Gemäß Beispiel 1 werden 115,4 g (0,79 mol) Adipinsäure, 218,3 g (1,73 mol) 2-Hydroxymethylnorbornan, 4,2 g (0,022 mol) p-Toluolsulfonsäure und 50 g Toluol zur Reaktion gebracht. Innerhalb von 2 Stunden werden 28,2 g Wasser ausgekreist. Das Reaktionsgemisch wird auf Raumtemperatur abgekühlt und mit 88,3 g wässriger Natronlauge (1 %ig) versetzt. Nach Phasentrennung verbleiben 353,8 g organische Phase, die anschließend mit 241,7 g Wasser gewaschen wird. Nach erneuter Phasentrennung wird die organische Phase (366,8 g) fraktioniert destilliert. Bei einer Kopftemperatur von 184 - 186°C und einem Druck von 100 Pa wird der Ester (247,6 g) in einer Reinheit von 98,9 % isoliert. Dies entspricht einer Ausbeute von 85,5 % der Theorie.

### Beispiel 5

### Herstellung von Di-(Norborn-2-yl-methyl)-sebacinsäureester

Gemäß Beispiel 1 werden 159,8 g (0,79 mol) Sebacinsäure, 218,3 g (1,73 mol) 2-Hydroxymethylnorbornan, 4,2 g (0,022 mol) p-Toluolsulfonsäure und 50 g Toluol zur Reaktion gebracht. Innerhalb von 2 Stunden werden 29,1 g Wasser ausgekreist. Das Reaktionsgemisch wird auf Raumtemperatur abgekühlt und mit 13,5 g wässriger Natronlauge (10 %ig) versetzt. Nach Phasentrennung verbleiben 404,7 g organische Phase, die anschließend dreimal mit insgesamt 682,0 g Wasser gewaschen wird. Nach erneuter Phasentrennung wird die organische Phase (381,8 g) fraktioniert destilliert. Bei einer Kopftemperatur von 220 - 222°C und einem Druck von 100 Pa wird der Ester (281,2 g) in einer Reinheit von 98,9 % isoliert. Dies entspricht einer Ausbeute von 84,1 % der Theorie.

## Patentansprüche

1. Carbonsäureester der allgemeinen Formel 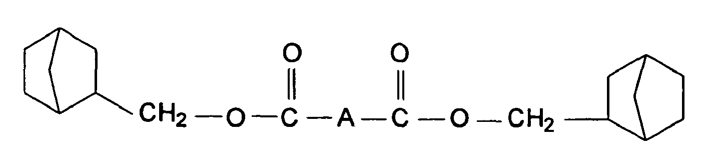 in der A gleich -(CH₂)ₓ-, mit x = 1 - 10 bedeutet.

2. Carbonsäureester nach Anspruch 1, **dadurch gekennzeichnet, dass** x gleich 1, 2, 3, 4, 7, 8 oder 10 bedeutet.

3. Verfahren zur Herstellung der Carbonsäureester gemäß Anspruch 1 durch Umsetzung von 2-Hydroxymethylnorbornan mit Dicarbonsäuren der allgemeinen Formel 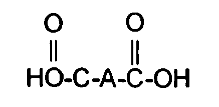 oder deren Anhydriden der allgemeinen Formel 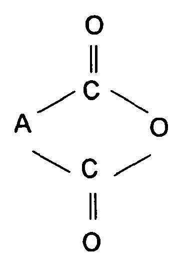 wobei A die in Anspruch 1 genannte Bedeutung besitzt, in Gegenwart eines Schleppmittels zur Entfernung des im Verlauf der Umsetzung gebildeten Wassers als azeotropes Gemisch und gegebenenfalls in Gegenwart eines Katalysators, Abtrennung überschüssiger und nicht umgesetzter Ausgangsstoffe, Behandlung mit einem alkalischen Reagenz zur Entfernung acider Bestandteile, gegebenenfalls anschließende Wasserdampfdestillation, und abschließende Trocknung und/oder fraktionierte Destillation.

4. Verwendung der Carbonsäureester gemäß Anspruch 1 als Schmiermittel oder Weichmacher für thermoplastische Kunststoffe.

## Claims

1. A carboxylic ester of the formula 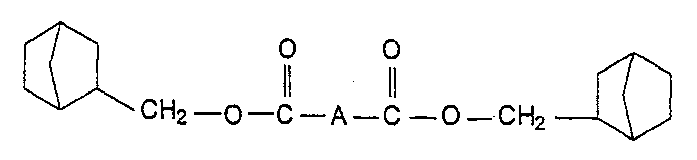 where A is -(CH₂)ₓ-, where x = from 1 to 10.

2. The carboxylic ester as claimed in claim 1, wherein x is 1, 2, 3, 4, 7, 8, or 10.

3. A process for preparing the carboxylic esters as claimed in claim 1 via reaction of 2-hydroxymethylnorbornane with dicarboxylic acids of the formula 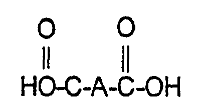 or with their anhydrides of the formula 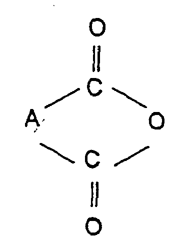 where A is as defined in claim 1, in the presence of an entrainer for removal, in the form of an azeotropic mixture, of the water formed during the course of the reaction, and, where appropriate, in the presence of a catalyst, removal of excess and unconverted starting materials, treatment with an alkaline reagent for removal of acidic constituents, and then, where appropriate, steam distillation, followed by drying and/or fractional distillation.

4. The use of the carboxylic esters as claimed in claim 1 as lubricants or plasticizers for thermoplastics.

## Revendications

1. Esters d'acides carboxyliques de formule générale 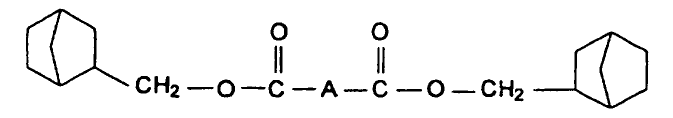 dans laquelle A représente -(CH₂)ₓ-, avec x = 1-10.

2. Esters d'acides carboxyliques selon la revendication 1, **caractérisés en ce que** x vaut 1, 2, 3, 4, 7, 8 ou 10.

3. Procédé pour la préparation des esters d'acides carboxyliques selon la revendication 1 par transformation de 2-hydroxyméthylnorbornane avec des acides dicarboxyliques de formule générale 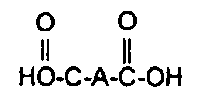 ou leurs anhydrides de formule générale 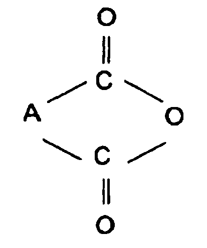 A présentant la signification mentionnée dans la revendication 1, en présence d'un agent d'entraînement pour éliminer l'eau formée au cours de la transformation sous forme d'un mélange azéotrope et le cas échéant en présence d'un catalyseur, séparation des substances de départ en excès et non transformées, traitement avec un réactif alcalin pour éliminer les constituants acides, le cas échéant distillation consécutive à la vapeur d'eau et séchage consécutif et/ou distillation fractionnée consécutive.

4. Utilisation des esters d'acides carboxyliques selon la revendication 1 comme lubrifiant ou plastifiant pour des matériaux synthétiques thermoplastiques.
